# EUROPEAN PATENT SPECIFICATION

(11) **EP 2 424 614 B1**
(45) Date of publication and mention of the grant of the patent: **23.04.2014**
(21) Application number: 09843809.6
(22) Date of filing: 01.05.2009
(51) Int. Cl.: A61N 1/04, A61B 5/0478, A61M 1/08, A61B 5/0424, H01R 13/717, A61B 5/06

(54) **INTEGRATED ELECTRODE CONNECTOR AND IMPEDANCE INDICATOR**
INTEGRIERTER ELEKTRODENKONNEKTOR UND IMPEDANZHEMMER
CONNECTEUR D'ELECTRODE INTEGRE ET INDICATEUR D'IMPEDANCE

(43) Date of publication of application: 07.03.2012
(73) Proprietor: Compumedics Limited, Abbotsford, Victoria 3067 (AU)
(72) Inventor: NEWMAN, Richard, Templestowe Victoria 3106 (AU); FREEMAN, Warwick, Abbotsford Victoria 3067 (AU)
(74) Representative: Handsome I.P. Ltd
(86) International application number: PCT/AU2009/000536
(87) International publication number: WO 2010/124317

(56) References cited:
- WO-A2-2006/097085
- US-A- 5 042 498
- US-A- 5 479 934
- US-A- 5 868 670
- US-A1- 2003 083 729
- US-A1- 2007 106 169
- US-B1- 6 971 907

## Description

### Field of the Invention

This invention relates to an electrode connector, in particular, an electrode connector in communication with an impedance monitor

### Background

There are many applications which use electrodes for making measurements of electrophysiological signals at the surface of the skin, such as for measuring brainwaves (EEG) or heart rate (ECG). The accuracy and precision of such electrophysiological signal measurements are dependent on the strength and reliability of the measured signal. Electrode-to-skin contact impedances are routinely measured to determine if an electrode is making good contact with the skin so that signal measurements are representative of the underlying electrophysiological activity.

It is known in the art to use single connectors for electrodes commonly used in medical applications, such as EEG or ECG as described in the DIN 42802 standard. On multi-electrode devices such as EEG an illuminated indicator is often positioned adjacent the electrode connector with which it is in electrical communication to indicate if the electrode impedance is outside of a preset value or range. For example, a green indicator may be illuminated to show that the electrode impedance is satisfactory or another indicator of another colour, typically orange, may be illuminated to show the electrode impedance is unacceptably high.

Where there are many electrodes, each with an impedance indicator, such as with apparatus for measuring EEG, the space available for impedance indicators is restricted. Close placement of electrodes and their attendant impedance indicators may make it difficult to determine which impedance indicator is in communication with a particular electrode, thus making the task of repairing the status of the electrode difficult. What is needed is an impedance indicator that shows whether or not an electrode is operating effectively but takes up relatively little space.

US6971907 discloses a connector for receiving an electrical plug having an at least partially transparent housing, and a light source 350 provided within the housing that emits a light in response to a sensed impedance. US2003/0083729 relates to a tester for electrode pads before they are removed from their packet. WO2006097085 relates to a light source located adjacent an electrode for measuring biopotentials. US5042498 relates to an ECG electrode having an LED associated with it.

### Brief Description of the Drawings

Figure 1 shows a perspective surface view of an integrated electrode connector and impedance indicator.
Figure 2 shows a longitudinal cross section of an integrated electrode connector and impedance indicator.
Figure 3 shows a side perspective view of an integrated electrode connector and impedance indicator with an electrode connector poised for engagement.

### Summary of the Invention

Currently there is no apparatus which incorporates an electrode connector and an impedance indicator with which it is in electrical communication. An aspect of the present invention may be found in the appendent independent claim 1, to which reference should now be made. Embodiments of the present invention may be found in the appendent dependent claims. The present invention provides apparatus that incorporates the function of both an electrode connector and an impedance indicator means in a single device. The invention provides apparatus comprising of an electrode connector, an impedance indicator means, and a light-transmitting housing. Preferably, the apparatus incorporates a locating means for locating the apparatus on an engagement means for anchoring the housing. Preferably, the engagement means is a suitable circuit board. Preferably the locating means is a post. The impedance indicator may be located in a recess in the housing. Alternatively, the impedance indicator could be located on the surface of the housing in a suitable location. The impedance indicator may be an LED or some other suitable light-emitting device. Preferably, the impedance indicator may indicate whether or not the impedance is inside or outside a particular desired operational range. Preferably, the impedance indicator indicates the status of the impedance being outside the operational range by emitting light. Alternatively, the impedance indicator could cease emitting light when the impedance was outside the operational range. Alternatively, the impedance indicator could change the colour of emitted light when the impedance was outside the operational range. The invention uses transparent or semi-transparent materials for the housing of the electrode connector and the impedance indicator means. The incorporation of the two signal measuring means into a single apparatus advantageously saves considerable space in multi-electrode devices, such as EEGs. It also advantageously provides an unambiguous indication whether a particular electrode connection is operating properly. This is a significant advantage over the prior art in situations in which it is not clear to which electrode connector an impedances indicator means is drawing attention because of the limited space between adjacent electrode connectors and associated impedance indicator means. The prior art may give rise to situations where it is not clear whether an indicator means above, below, or to one side of an electrode connector is indicating that electrode connector is not functioning properly.

### Detailed Description of the Figures and Best Method of Performance

The invention is illustrated in one embodiment in the accompanying figures. It will be understood by those skilled in the art that the invention is not limited to the embodiment illustrated in the figure but instead includes any embodiment within the scope of the claims appended hereto.

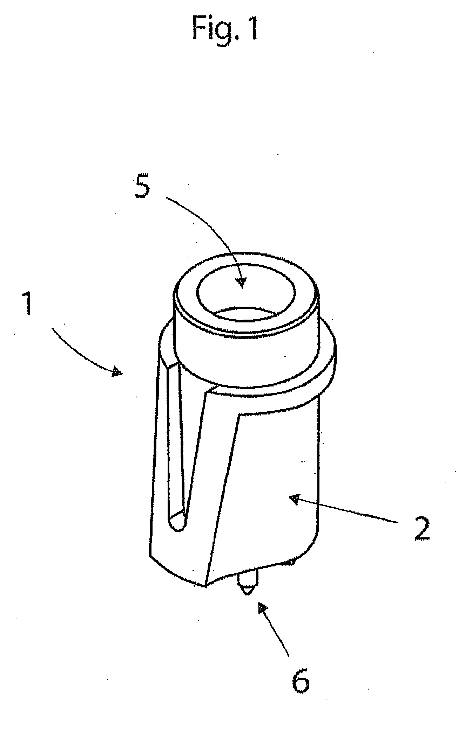

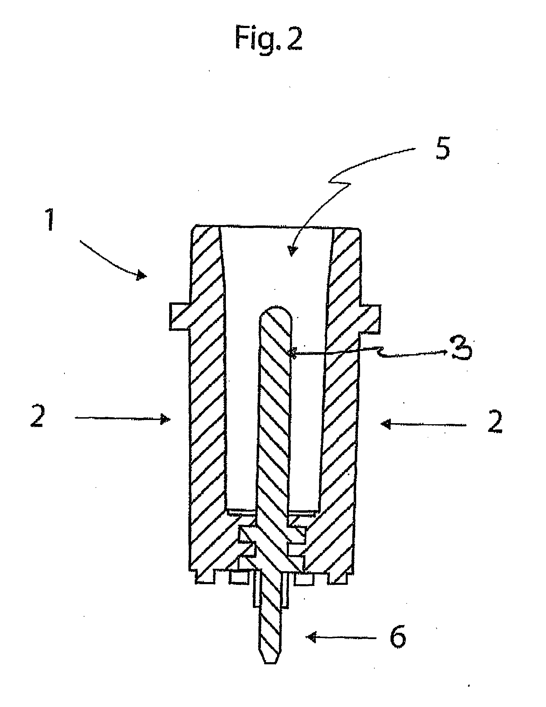

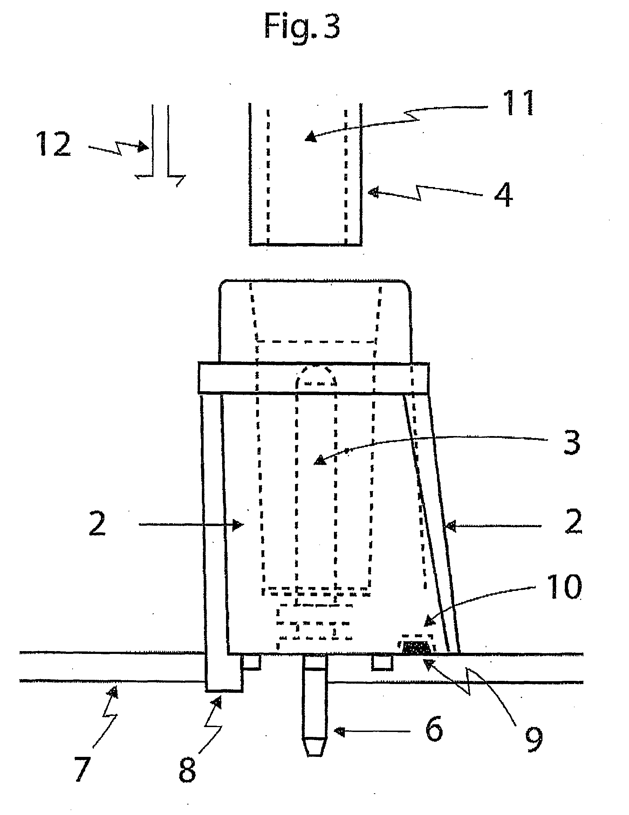

Figure 1 shows an embodiment of an electrode connector incorporating an impedance indicator means according to the invention. In Figure 1 the integrated electrode connector and impedance indicator 1 comprises of a light-transmitting housing 2 having a conduit 5 for engaging an electrode lead wire connector. Preferably, the housing 2 transmits light. In Figure 2 is shown in transverse longitudinal section the embodiment of the integrated electrode connector 1 of Figure 1. The integrated electrode connector incorporates a conducting pin 3 for engaging with the electrode lead wire connector 4. The conducting pin 3 includes a distal end 6 for engaging with a conducting circuit on an engagement means 7, such as a circuit board as shown in Figure 3. Figure 3 shows a side view of an embodiment of an integrated electrode connector and impedance indicator 1 and an electrode lead wire connector 4. The electrode lead wire connector incorporates a channel 11 for conductively engaging the conducting pin 3 when engaged with the integrated electrode connector 1 in the direction of the arrow 12.

Preferably, the conducting pin 3 has a diameter of approximately 1.5 mm to meet the specification of the DIN 42802 standard, which is known in the art as the standard for electrode connectors. The electrode lead wire connector may be any suitable connector.

Preferably, the housing 2 is comprised of transparent material. Alternatively, the material may be translucent, semi-transparent or another light-transmitting characteristic, as long as it is capable of transmitting light from an indicating means.

The housing forms a conduit 5 for guiding the electrode lead wire connector 4 for engaging the conducting pin 3.

The engagement means 7 functions to anchor the housing 2, conducting pin 3, and circuit (not shown) so that a complete circuit for conducting electrophysiological signals can be transmitted for processing. The engagement means 7 must be in communication with an impedance-monitoring circuit.

The housing 2 may include locating means 8 for locating the housing on the engagement means 7 to guide and position the housing 2 correctly on the engagement means 7 so that the conducting pin 3 makes a conducting engagement with the circuit. Preferably, the engagement means 7 may be any type of circuit board.

Preferably the locating means 8 is a post. Most advantageously, adjacent the housing 2 is an indicating means 9 for indicating that the impedance of the electrode is within the specified range. The indicating means 9 may be made of any material that generates or conducts light. Preferably the indicating means is an LED.

By placing the indicating means adjacent the light-transmitting housing, any light that is conducted or generated by the indicating means may be transmitted through the light-transmitting housing for indicating the status of the electrical connection between the electrode lead connector 4 and circuit to a user. Preferably the indicating means 9 is accommodated by a recess 10 in the housing 2. This preferred arrangement allows an indicating means such as an LED to be accommodated in the recess 10.

In operation, the state of the indicator means draws attention to the impedance of the electrode connector. In one state of the indicator means, the impedance of the electrode is within the desired operating range for the transmission of electrophysiological signals. Preferably, the indicator means is illuminated to indicate that the electrode impedance is outside of it operational range. Preferably the indicator means is an LED. Preferably the LED is visibly illuminated when the electrode lead is in electrical communication with the connector.

## Claims

1. Apparatus (1) comprising of:
an electrophysiological skin surface electrode connector for use with an electrode for making measurements of electrophysiological signals at a skin surface;
a light-transmitting housing (2); and
an impedance indicator means (9) configured to be in a first state in response to an electrode-to-skin contact impedance of an electrode (4) coupled to the electrode connector being within a specific operating range for the transmission of electrophysiological signals, and configured to be in a second state in which the indicator means conducts or generates light in response to the impedance of the electrode being outside the specific operating range;
wherein the impedance indicator means (9) is arranged adjacent the light-transmitting housing (2) such that light conducted or generated by the impedance indicator means (9) is transmitted through the light-transmitting housing (2).

2. The apparatus of claim 1, wherein the impedance indicator means (9) is located in a recess.

3. The apparatus of any one of claims 1 or 2, wherein the impedance indicator means (9) is an LED.

4. The apparatus of any one of claims 1 to 3, wherein the electrode connector incorporates a conducting pin (3) for engaging with an electrode lead wire (4).

5. The apparatus of claim 4, wherein the conducting pin (3) has a diameter of approximately 1.5mm to meet the specification of the DIN 42802 standard.

6. The apparatus according to any one of claims 1 to 5, further comprising location means (8) for locating the housing.

7. The apparatus according to claim 6 wherein the location means (8) is a post.

8. The apparatus according to any one of claims 1 to 7, further comprising engagement means (7) for anchoring the housing.

9. The apparatus of claim 8, wherein the engagement means (7) is a circuit board.

10. The apparatus of any preceding claim, wherein the electrode connector is configured to connect to an EEG electrode.

## Patentansprüche

1. Gerät (1) umfassend:
einen elektrophysiologischen Hautoberflächenelektroden-Verbinder für die Verwendung mit einer Elektrode zur Durchführung von Messungen elektrophysiologischer Signale an einer Hautoberfläche;
ein lichtdurchlässiges Gehäuse (2); und
Impedanzanzeigemittel (9), so ausgebildet, dass sie in einem ersten Zustand eine Antwort auf eine Elektrode-zu-Haut-Kontaktimpedanz einer Elektrode (4) geben, die mit dem Elektrodenverbinder gekoppelt ist, der sich in einem spezifischen Arbeitsbereich befindet für den Durchlass elektrophysiologischer Signale und ausgebildet, um in einem zweiten Zustand, in dem die Indikatormittel Licht leiten oder erzeugen, eine Antwort auf die Impedanz der Elektrode zu geben, die sich außerhalb des spezifischen Arbeitsbereichs befindet;
wobei die Impedanzanzeigemitel (9) benachbart zu dem lichtdurchlässigen Gehäuse (2) angeordnet sind, so dass von den Impedanzanzeigemitteln (9) geleitetes oder erzeugtes Licht durch das lichtdurchlässige Gehäuse (2) durchgelassen wird.

2. Gerät nach Anspruch 1, bei dem die Impedanzanzeigemittel (9) in einer Vertiefung angeordnet sind.

3. Gerät nach einem der Ansprüche 1 oder 2, bei dem die Impedanzanzeigemittel (9) eine LED sind.

4. Gerät nach einem der Ansprüche 1 bis 3, bei dem der Elektrodenverbinder einen leitenden Stift (3) umfasst für eine Verbindung mit einem Elektrodenführungsdraht (4).

5. Gerät nach Anspruch 4, bei dem der leitende Stift (3) einen Durchmesser von etwa 1,5 mm aufweist, um die Spezifikation des DIN 42802 Standards zu erfüllen.

6. Gerät nach einem der Ansprüche 1 bis 5, weiterhin umfassend Platzierungsmittel (8) für die Platzierung des Gehäuses.

7. Gerät nach Anspruch 6, bei dem die Platzierungsmittel (8) ein Pfosten sind.

8. Gerät nach einem der Ansprüche 1 bis 7, welches weiterhin Eingriffsmittel (7) umfasst für die Verankerung des Gehäuses.

9. Gerät nach Anspruch 8, bei dem die Eingriffsmittel (7) eine Leiterplatte sind.

10. Gerät nach einem der vorhergehenden Ansprüche, bei dem der Elektrodenverbinder ausgebildet ist für die Verbindung mit einer EEG-Elektrode.

## Revendications

1. Appareil (1) comprenant :
un connecteur d'électrode de surface de peau électrophysiologique pour une utilisation avec une électrode pour effectuer des mesures de signaux électrophysiologiques au niveau d'une surface de peau ;
un boîtier transmettant la lumière (2) ; et
un moyen indicateur d'impédance (9) configuré pour être dans un premier état en réponse à une impédance de contact électrode-peau d'une électrode (4) couplée au connecteur d'électrode qui est dans une plage de fonctionnement spécifique pour la transmission de signaux électrophysiologiques, et configuré pour être dans un second état dans lequel le moyen indicateur conduit ou génère de la lumière en réponse à l'impédance de l'électrode qui est en dehors de la plage de fonctionnement spécifique ;
le moyen indicateur d'impédance (9) étant agencé adjacent au boîtier transmettant la lumière (2) de telle sorte que la lumière conduite ou générée par le moyen indicateur d'impédance (9) est transmise à travers le boîtier transmettant la lumière (2).

2. Appareil selon la revendication 1, dans lequel le moyen indicateur d'impédance (9) est situé dans un renfoncement.

3. Appareil selon l'une quelconque des revendications 1 ou 2, dans lequel le moyen indicateur d'impédance (9) est une DEL.

4. Appareil selon l'une quelconque des revendications 1 à 3, dans lequel le connecteur d'électrode incorpore une broche conductrice (3) destinée à s'engager avec un fil conducteur d'électrode (4).

5. Appareil selon la revendication 4, dans lequel la broche conductrice (3) a un diamètre d'approximativement 1,5 mm pour satisfaire aux exigences de la norme DIN 42802.

6. Appareil selon l'une quelconque des revendications 1 à 5, comprenant en outre un moyen de positionnement (8) pour positionner le boîtier.

7. Appareil selon la revendication 6, dans lequel le moyen de positionnement (8) est un montant.

8. Appareil selon l'une quelconque des revendications 1 à 7, comprenant en outre un moyen d'engagement (7) pour ancrer le boîtier.

9. Appareil selon la revendication 8, dans lequel le moyen d'engagement (7) est une carte de circuit.

10. Appareil selon l'une quelconque des revendications précédentes, dans lequel le connecteur d'électrode est configuré pour se connecter à une électrode d'EEG.
